# EUROPEAN PATENT APPLICATION

(11) **EP 4 438 712 A1**
(43) Date of publication of application: **02.10.2024**
(21) Application number: 22899053.7
(22) Date of filing: 24.11.2022
(51) Int. Cl.: C12M 3/00, C12M 1/12, C12M 3/06, C12N 5/09

(54) **TUMOR TISSUE MODEL FOR CULTURING TUMOR TISSUE**

(30) Priority: 24.11.2021 KR 20210163531
(71) Applicant: Industry-Academic Cooperation Foundation, Yonsei University, Seoul 03722 (KR)
(72) Inventor: KANG, Seok-Gu, Suwon-si Gyeonggi-do 16517 (KR); SUNG, Hak-Joon, Seoul 06083 (KR); BAEK, Se Woom, Seoul 03721 (KR)
(74) Representative: Schiweck Weinzierl Koch Patentanwälte Partnerschaft mbB
(86) International application number: PCT/KR2022/018714
(87) International publication number: WO 2023/096371

(57) **Abstract**

The present invention relates to a tumor tissue model in which tumor tissue isolated from a patient can be cultured. The tumor tissue model uses tumor tissue isolated from a patient, and thus the tumor tissue can be cultured while maintaining the extracellular matrix of tumor cells. Since the tumor tissue is cultured in an environment similar to the actual environment inside the body, the tumor tissue model can be used for patient-tailored drug screening, drug response evaluation, radiation reactivity evaluation, tumor-related research, clinical evaluation, animal replacement tests, disease modeling, etc.

## Description

### [Technical Field]

The present invention relates to a tumor tissue model in which tumor tissue isolated from a patient may be cultured in an environment and conditions similar to those in actual living organisms.

### [Background Art]

A brain tumor refers to a tumor that originates in brain tissue or the membrane surrounding the brain and a tumor that has metastasized to brain tissue or meninges from a position far away from the skull or surrounding structures. Brain tumors may be classified into benign brain tumors, which have a slow growth rate and have clear boundaries with surrounding tissue and thus may be removed by surgery, and malignant brain tumors, which have a fast growth rate and have a strong ability to infiltrate surrounding tissue and thus are difficult to treat. Malignant brain tumors are also referred to as brain cancer.

The five-year survival rate for all brain tumor patients exceeds 65%, and most benign brain tumors such as meningiomas and pituitary tumors are fully cured by complete resection. However, the five-year survival rate of malignant brain tumor patients is only 22% to 33% on average, and glioblastoma (GBM), which is classified as the most malignant cancer, has very high resistance to radiotherapy and chemotherapy compared to other cancers, so once diagnosed, the patients' survival period is only one year.

For brain tumors, tumor excision is the most effective treatment method when possible, and this method is often used to treat benign tumors. However, malignant brain tumors are very likely to have cancer cells remaining in other regions even after tumor excision, so additional treatment such as radiotherapy and chemotherapy is required.

Meanwhile, personalized medicine has recently been attempted in which treatment methods vary depending on each patient's expected drug response and sensitivity. For this, in the field of brain tumor treatment, drug testing is conducted by isolating patient-derived tumor cells, and drug responsiveness is verified through patient-derived xenografts in which tumor cells are transplanted into an animal model. However, since it takes a long time, more than six months, to verify drug responsiveness, it is not practical for malignant brain tumor patients, whose survival rate is low. In addition, the existing patient-derived cell culture method involves isolating and culturing tumor cells, which results in the loss of extracellular matrix, and xenograft-based animal models lose a tumor's unique pathological characteristics, so it is difficult to expect the treatment to be actually effective in patients.

There is an organ simulation chip (organ-on-a-chip) that simulates the functions of the human body by mixing two-dimensionally cultured single cells or co-cultured multiple cells and then inserting them into a hydrogel or scaffold, but it is difficult to fully reflect the actual characteristics of the human body. In particular, organoid culture has an extremely low culture success rate, and it requires enormous economic consumption and inefficient culture time compared to general cell culture.

Therefore, unlike existing tumor cell culture methods or organ simulation chips, there is a need for a tumor cell or tumor tissue culture method that is capable of reflecting the actual characteristics of the human body while maintaining the extracellular matrix of tumor cells.

### [Disclosure]

### [Technical Problem]

In above-described situation, the present inventors confirmed that when tumor tissue isolated by biopsy from a tumor patient was cultured in a structure for tissue culture made of a biocompatible polymer and including a three-dimensional network formed by connecting a plurality of microchannels therein, the tumor tissue was cultured in an environment that was similar to the *in vivo* environment of the tumor, and the survival rate of the tumor tissue was maintained, thereby completing the present invention.

Therefore, an object of the present invention is to provide a tumor tissue model capable of culturing patient-derived tumor tissue and a method for producing the same.

### [Technical Solution]

One aspect of the present invention provides a tumor tissue model including:
a structure for tissue culture made of a biocompatible polymer and including a three-dimensional network formed by connecting a plurality of microchannels therein; and
tumor tissue isolated from the living body,
wherein the tumor tissue is encapsulated in the structure for tissue culture.

The term "biocompatible polymer" used in the present specification refers to a polymer having tissue compatibility and blood compatibility that does not destroy tissue or coagulate blood when coming into contact with living tissue or blood.

In the present invention, the biocompatible polymer may be selected from the group consisting of gelatin, alginate, polyethylene glycol (PEG), polydimethylsiloxane (PDMS), collagen, chitin, chitosan, keratin, cellulose, fibronectin, elastin, fibrinogen, fibromodulin, laminin, tenacin, vitronectin, hyaluronic acid, heparin, collagen, chondroitin sulfate, pectin, dextran, silk protein, silk fibroin and derivatives thereof, agarose, polylactic acid (PLA), polyglycolic acid (PGA), copolymers of polylactic acid and polyglycolic acid (PLGA), polycaprolactone (PCL), poly(poly(ethylene oxide)terephthalate-co-butylene terephthalate) (PEOT/PBT), polyphosphoester (PPE), polyphosphazene (PPA), polyanhydride (PA), polyorthoester (POE), and polypropylene fumarate)-diacrylate (PPF-DA), and preferably, alginate, gelatin, PDMS, or PEG may be used.

The present inventors studied a structure which has a network structure similar to the *in vivo* vascular structure and which is capable of culturing tumor tissue, and produced a tissue culture structure in which a fluid may flow through an internal three-dimensional microchannel network to form a circulatory system.

According to one embodiment of the present invention, the microchannel may have a diameter in the range of 5 to 50 µm, preferably 10 to 50 µm, more preferably 10 to 30 µm. In the present invention, the hydrogel structure may further include an inflow channel and an outflow channel, and the inflow channel and the outflow channel are connected to microchannels to form a circulatory system through which a fluid flows.

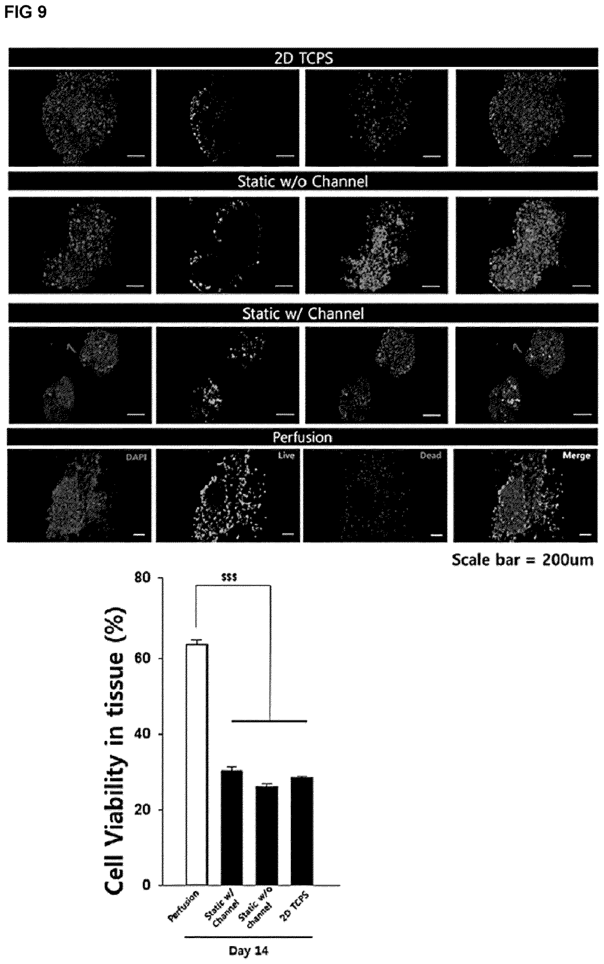

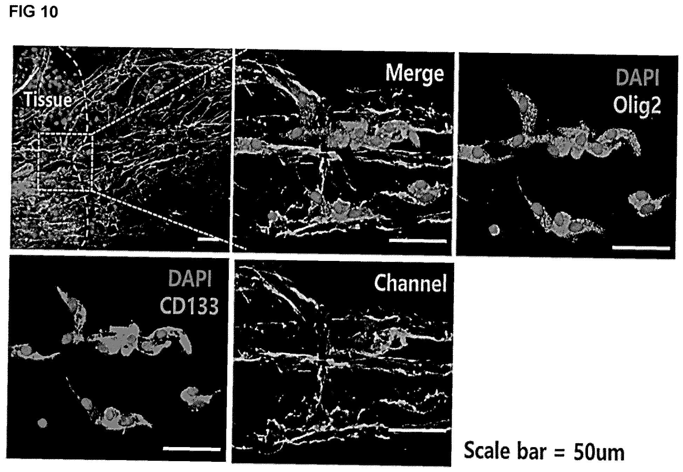

In addition, the present inventors produced a tissue culture structure including patient-derived brain tumor tissue having a size of less than 1 mm, and as a result of culturing the brain tumor tissue while perfusing a cell culture medium, it was confirmed that the cell survival rate was maintained, and brain cancer cells moved into the microchannels (FIGS. 9 and 10). These results mean that the tissue culture structure of the present invention provides an environment that is similar to the actual *in vivo* environment for tumor tissue.

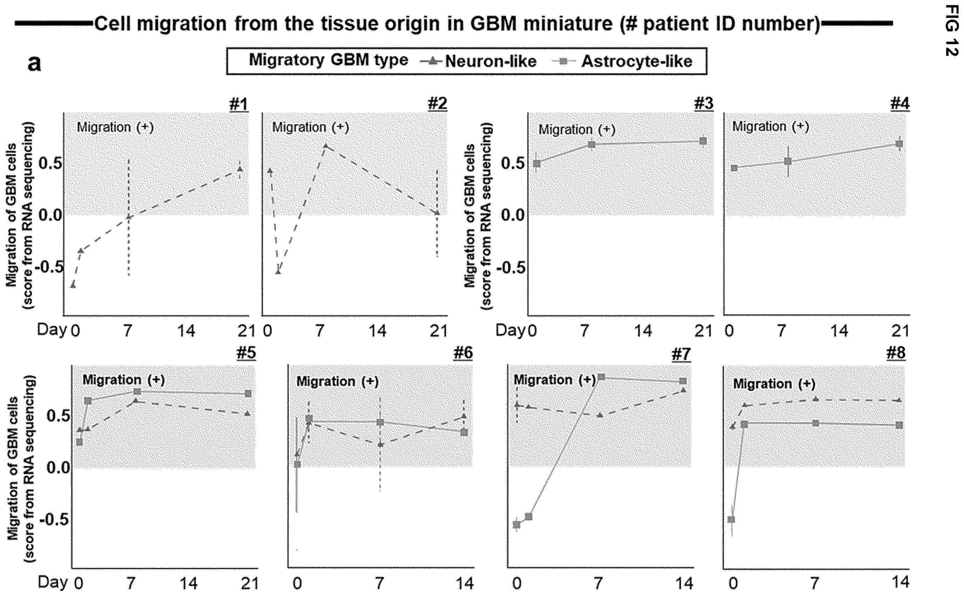

In addition, since brain cancer cells have a strong ability to metastasize and migrate through blood vessels and nerves, brain cancer is difficult to treat and has high recurrence and mortality rates. The results shown in FIGS. 10 and 12 mean that the tumor tissue model of the present invention may reproduce the *in vivo* characteristics of the highly mobile brain cancer cells at the laboratory level.

Therefore, the tumor tissue, while not being limited thereto, may be selected from the group consisting of brain tumors, stomach cancer, liver cancer, colon cancer, breast cancer, kidney cancer, prostate cancer, cervical cancer, endometrial cancer, and ovarian cancer. Cervical cancer, endometrial cancer, and ovarian cancer are representative gynecological cancers, and their incidence rates have recently been increasing among women in their 20s and 30s.

According to one embodiment of the present invention, the tumor tissue may be a brain tumor, and preferably, may be glioblastoma.

A tumor tissue model according to one embodiment of the present invention is produced by a method including the following steps:
(a) supplying a hydrogel solution containing tumor tissue to a mold containing a three-dimensional fiber bundle;
(b) crosslinking the hydrogel solution; and
(c) removing the three-dimensional fiber bundle from the crosslinked hydrogel.

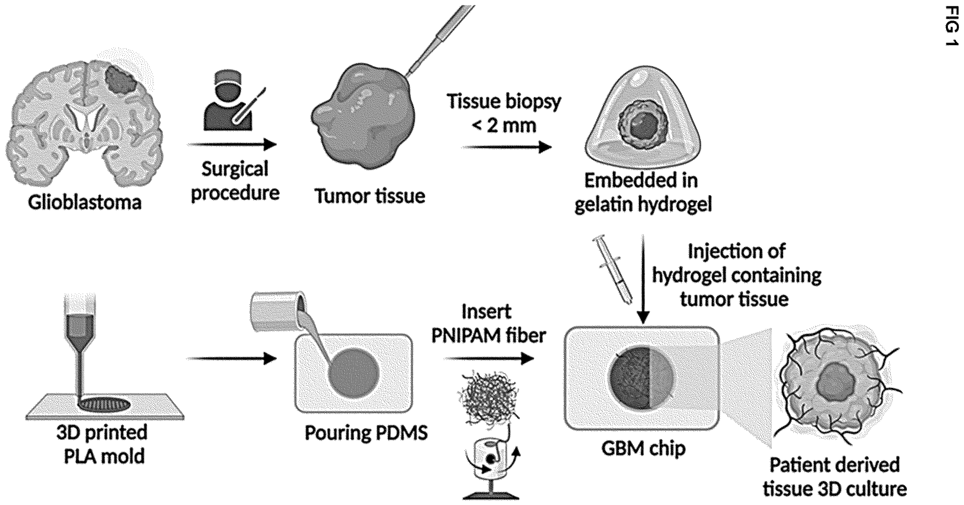

According to one embodiment of the present invention, a method for producing a tumor tissue model includes two processes: isolating tumor tissue from a patient; and producing a structure for tissue culture, as illustrated in FIG. 1.

The process of producing a structure for tissue culture begins with making a three-dimensional fiber bundle made of a temperature-sensitive polymer. A three-dimensional fiber bundle forming a microchannel is manufactured by spinning a stimuli-responsive polymer solution using a rotary spinning device, and the fiber diameter may be adjusted by changing the rotation speed.

The term "stimuli-responsive polymer" used in the present specification refers to a polymer whose physical properties such as sol-gel phase transition or volume phase transition are changed reversibly or irreversibly by stimuli such as temperature, pH, electricity, stress, pressure, and magnetism, and it is referred to as a "temperature-responsive polymer (TRP)" when the stimulus is temperature. Polymers exhibiting lower critical solution temperature (LCST) behavior are in a sol state at temperatures lower than an LCST.

According to one embodiment of the present invention, the temperature-responsive polymer may be selected from the group consisting of poly(N-isopropyl acrylamide), poly-N-vinylcaprolactam, poly(methacrylic acid), hydroxypropyl cellulose, polyvinyl methyl ether, poly(2-hydromethacrylic acid), poly(N-isopropylacrylamide), poly(N,N-diethylacrylamide), poly(N-diethyl methacrylamide), poly(methyl vinyl ether), poly(2-ethoxyethyl vinyl ether), poly(N-vinyl caprolactam), poly(N-vinyl isobutylamine), poly(N-vinyl-n-butylamine), and polyfumaric acid, and preferably, poly(N-isopropyl acrylamide) may be used.

According to one embodiment of the present invention, the poly(N-isopropyl acrylamide) has a number average molecular weight of 85,000, is dissolved in methanol at a concentration of 34% to 36% to prepare a solution, which is then spun to produce a three-dimensional fiber bundle.

By using poly(N-isopropylacrylamide) having the above molecular weight, the temperature maintenance ability of the structure for tissue culture (hydrogel) was increased, and the holding power to suppress the pushing phenomenon of the channel structure, which occurs during hydrogel injection, could be obtained.

According to one embodiment of the present invention, the hydrogel crosslinking in (b) may be performed using both physical and chemical crosslinking methods, and for example, an enzymatic crosslinking agent such as transglutaminase may be used. Enzymatic crosslinking agents are inexpensive, biocompatible, and practical because they allow easy loading of cells and therapeutic agents prior to gelation. Furthermore, enzymatic crosslinking agents may be applied to a wide range of tissue and organs because the cross-linking time and degree of cross-linking may be controlled by adjusting the ratio of hydrogel solution to enzyme.

According to one embodiment of the present invention, the ratio of the hydrogel solution and transglutaminase may be in the range (w/v%) of 5:1 to 30:1, preferably 5:1 to 25:1.

In the present invention, (c) may be achieved by converting the three-dimensional fiber bundle into a sol, and specifically, fibers made of a temperature-responsive polymer, which are present as an aqueous solution at a temperature lower than an LCST, may be removed by perfusing a fluid at a temperature lower than the LCST into the hydrogel. Since the poly(N-isopropylacrylamide (PNIPAM; Mn=85,000) exhibits an LCST of about 32 °C, PNIPAM fibers may be removed by perfusing a fluid at a temperature lower than this temperature into a structure for tissue culture.

Another aspect of the present invention provides a tumor tissue culture method, including:
supplying a cell culture medium to the tumor tissue model; and
culturing the tumor tissue model.

According to one embodiment of the present invention, the cell culture medium may be supplied at a rate of 1 to 15 mm/s, which is similar to the blood flow rate in actual capillaries in the body, and it may be supplied at a rate of preferably 3 to 12 mm/s, more preferably 3 to 10 mm/s, and most preferably 5 to 7 mm/s.

Among the terms or elements mentioned in the tumor tissue culture method, those that are the same as mentioned in the description of the tumor tissue model are understood to be the same as mentioned in the description of the claimed tumor tissue model.

Meanwhile, another aspect of the present invention provides a patient-tailored drug screening method, a drug responsiveness evaluation method, and a radiation reactivity evaluation method including the tumor tissue model.

### [Advantageous Effects]

The tumor tissue model according to the present invention uses tumor tissue isolated from a patient, and thus the tumor tissue can be cultured while maintaining the extracellular matrix of tumor cells. Since the tumor tissue is cultured in an environment similar to the actual environment inside the body, the tumor tissue model can be used for patient-tailored drug screening, drug response evaluation, radiation reactivity evaluation, tumor-related research, clinical evaluation, animal replacement tests, disease modeling, etc.

### [Description of Drawings]

FIG. 1 schematically shows a process of manufacturing a tumor tissue model according to an example of the present invention.

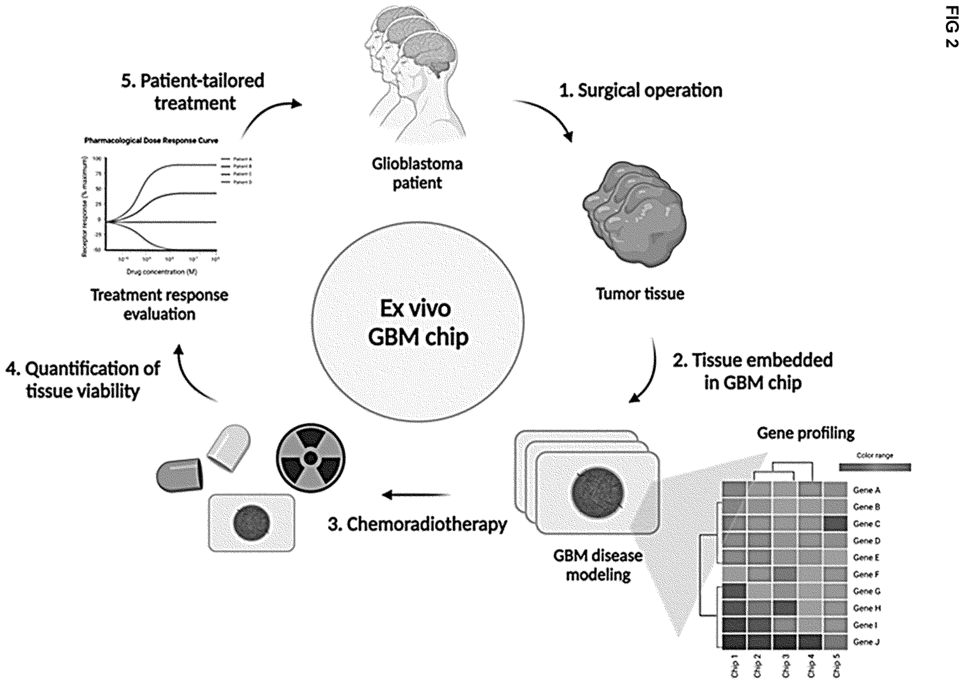

FIG. 2 shows the utilization of a brain tumor tissue model according to an example of the present invention.
FIG. 3 shows the results of confirming a cell survival rate in brain tumor tissue isolated from a brain tumor patient.
FIG. 4 shows the results of confirming a cell survival rate by thawing brain tumor tissue isolated from a brain tumor patient after cryopreserving it for more than six months.
FIG. 5 shows the results of confirming the diameter of poly(N-isopropyl acrylamide) (PNIPAM) fibers manufactured by dissolving PNIPAM with a molecular weight of less than 40,000 in methanol at a concentration of 45 w/v%.
FIG. 6 shows the results of confirming the diameter of PNIPAM fibers manufactured by dissolving PNIPAM with a molecular weight of 85,000 in methanol at a concentration of 36 w/v%.
FIG. 7 shows the results of observing fibers produced by dissolving PNIPAM with a molecular weight of 85,000 in methanol at various concentrations and spinning the corresponding solutions.
FIG. 8 shows the results of confirming the survival rate of cells after culturing brain tumor tissue isolated from a brain tumor patient for 3 days in a tumor tissue model.
FIG. 9 shows the results of confirming the survival rate of cells after culturing brain tumor tissue isolated from a brain tumor patient for 14 days under various conditions: 2D TCPS - cultured in a two-dimensional cell culture dish; Static w/o channel - only brain tumor tissue is embedded in a gelatin hydrogel without microchannels and cultured without perfusion; Static w/ channel - brain tumor tissue is embedded in a gelatin hydrogel with microchannels and cultured without perfusion; and Perfusion - brain tumor tissue is embedded in a gelatin hydrogel with microchannels and cultured while perfusing a culture medium.
FIG. 10 shows the results that brain cancer cells cultured in a tumor tissue model enter the microchannel network structure.
FIG. 11 shows the results of comparing gene expression patterns between original brain tumor tissue derived from a patient and brain tumor tissue cultured in a tumor tissue model.
FIG. 12 shows the results of analyzing an expression pattern of migratory genes in brain tumor tissue cultured in a tumor tissue model.

### [Modes of the Invention]

Hereinafter, one or more embodiments will be described in more detail through examples. However, these examples are for the purposes of illustrating one or more embodiments and the scope of the present invention is not limited to these examples.

### Experimental method

### 1. Brain tumor tissue selection

Among the types of brain tumors, Grade IV glioblastoma (GBM) was classified through clinical examination. Tumor tissue was used within a maximum of one day after surgery, and it was moved and stored at 0 to 4 °C after surgical treatment to preserve the viability of the tumor tissue. The tissue culture medium was prepared by adding a 1x B27 supplement, a human fibroblast growth factor (FGF), and an epidermal growth factor (EGF), each at a concentration of 10 ng/ml, to 1x Dulbecco's Modified Eagle Medium/Nutrient Mixture F-12 (DMEM/F-12) (50/50) containing L-glutamine.

After tissue biopsy (< 2 mm), the tissue survival rate was confirmed using the Live/Dead kit (Thermofisher, L3224, Carlsbad, CA, USA). To confirm live cells, a calcein AM solution, which exhibits green fluorescence, was added at a concentration of 2 µl/ml, and to confirm dead cells, an ethidium homodimer-1 solution, which exhibits red fluorescence, was added at a concentration of 1 µl /ml. Afterward, the brain tumor tissue was cultured at 37 °C and 5% CO₂ for 30 min, washed three times with PBS, and photographed with a fluorescence microscope (Leica, DMi8, Wetzlar, Germany).

### 2. Cryopreservation of brain tumor tissue

Brain tumor tissue received after surgical operation was biopsied at a length, width, and height of less than 1 mm. The biopsied brain tumor tissue was placed in a cell cryopreservation vial, and a cryopreservation solution (CryoStor CS10, STEMCELL Tech, #07930, Van, Canada) was added at a level of 20 pcs (pieces)/ml of tissue to cryopreserve the brain tumor tissue. When thawing after cryopreservation, the brain tumor tissue was thawed as quickly as possible, placed in a tissue culture medium, and then spun down for use.

### 3. Production of brain tumor tissue model

A three-dimensional vascular microchannel network structure of the brain tumor tissue model was produced using a microvial transglutaminase (mTG)-based enzyme-crosslinkable gelatin hydrogel.

The microchannels were produced using thermosensitive poly(Nisopropyl acrylamide) (PNIPAM; Mn=85,000; Sigma-Aldrich, USA). PNIPAM was dissolved in methanol at a concentration of 36 w/v%, and the resulting solution was spun using a rotary spinning device to produce a PNIPAM fiber bundle.

The mTG was dissolved in phosphate buffered saline (PBS) at a concentration of 10% (w/v) and sterilized using a syringe filter. Gelatin (Sigma-Aldrich, G1890, USA) powder was dissolved in PBS at a concentration of 5.5% (w/v), completely dissolved by stirring (400 rpm) with a magnetic bar at 37 °C for 12 hours, and then the resulting solution was sterilized with a vacuum filter. The sterilized mTG solution and gelatin powder solution were stored in an oven at 50 °C.

The produced PNIPAM fiber bundle was placed in a polydimethylsiloxane (PDMS) mold, and a gelatin/mTG solution including brain tumor tissue (9:1 ratio, final concentration = 5 w/v%) was poured thereinto and the gelatin was cross-linked at 37 °C for 30 minutes. Afterward, silicone tubes were connected to both ends of the gelatin hydrogel, the temperature was lowered to room temperature below 32 °C to remove the PNIPAM fibers, and then PBS was perfused to completely remove the PNIPAM fibers.

Since the actual blood flow rate in capillaries in the body is 7.17 mm/s ± 2.35 mm/s, the culture medium was supplied at 6.33 mm/s (376.9 µl/min rate) during the brain tumor tissue culture, and the brain tumor tissue was cultured until the time to observe it.

FIG. 1 schematically shows the brain tumor tissue model production process.

### 4. Establishment of brain tumor tissue culture conditions

To confirm a suitable environment for culturing a patient's brain tumor tissue, the biopsied brain tumor tissue was cultured in a brain tumor tissue model under various conditions from 1 day to a maximum of 28 days. The culture conditions used were as follows: 2D TCPS - cultured in a two-dimensional cell culture dish; Static w/o channel - only brain tumor tissue is embedded in a gelatin hydrogel without microchannels and cultured without perfusion; Static w/ channel - brain tumor tissue is embedded in a gelatin hydrogel with microchannels and cultured without perfusion; and Perfusion - brain tumor tissue is embedded in a gelatin hydrogel with microchannels and cultured while perfusing a culture medium.

After culturing the brain tumor tissue for different periods of time under the above conditions, staining was performed (calcein AM/ethidium homodimer-2) to investigate the viability of the tissue, and the survival rate of the tissue was evaluated by observation with a fluorescence microscope.

### 5. Large-scale RNA analysis

The brain tumor tissue in the brain tumor tissue model was cultured for about 21 days and then recovered. RNA was extracted from the brain tumor tissue model, and total RNA gene expression was analyzed using a next-generation sequencing (NGS) method. The gene expression analysis results were aligned with the reference sequence, chromosome GRCh38.84, using the Linux Pipeline and HISAT2 (Version 2.1.0). The aligned gene sequences were calculated using the fragments per kilobase million (FPKM) method. To view changes in the gene set, gene expression scores were calculated in each sample using the gene set variation analysis (GSVA) algorithm (Version 1.42.0) and converted to the Human Genome Organization (HUGO) gene symbols stored in Ensembl. Genes corresponding to the gene set to be investigated (glioblastoma cells, dividing cells, neurons, immune cells, and vascular cells) were selected, and expression scores were comprehensively calculated and displayed in the form of heat maps and graphs.

### 6. Fluorescent immunostaining

The brain tumor tissue in the brain tumor tissue model was cultured for about 21 days and then recovered. The brain tumor tissue model was perfused with PBS three times, and then the brain tumor tissue was fixed with 4% paraformaldehyde at 4 °C for 24 hours. A the brain tumor tissue permeabilization process was performed by perfusing PBS containing a 0.5% Triton-X 100 solution. A 1% bovine serum albumin (BSA) solution was prepared with PBS containing 0.1% Triton-X100 and then perfused to block the brain tumor tissue for one hour.

Brain tumor markers CD133 and Olig2 were diluted in a 0.1% Triton-X100 solution at ratios of 1:100 and 1:200, respectively, and allowed to react with the brain tumor tissue at 4 °C for 24 hours. Afterward, PBS was perfused three times, and IgG anti-rabbit and anti-mouse antibodies conjugated with Alexa 488 and 594 were diluted 1:200 in PBS and allowed to react with the brain tumor tissue for two hours. After PBS was perfused three times, nuclear staining was performed, and the brain tumor cells cultured in the brain tumor tissue model were observed using a fluorescence microscope.

### Experiment results

### 1. Confirmation of survival rate of brain tumor tissue

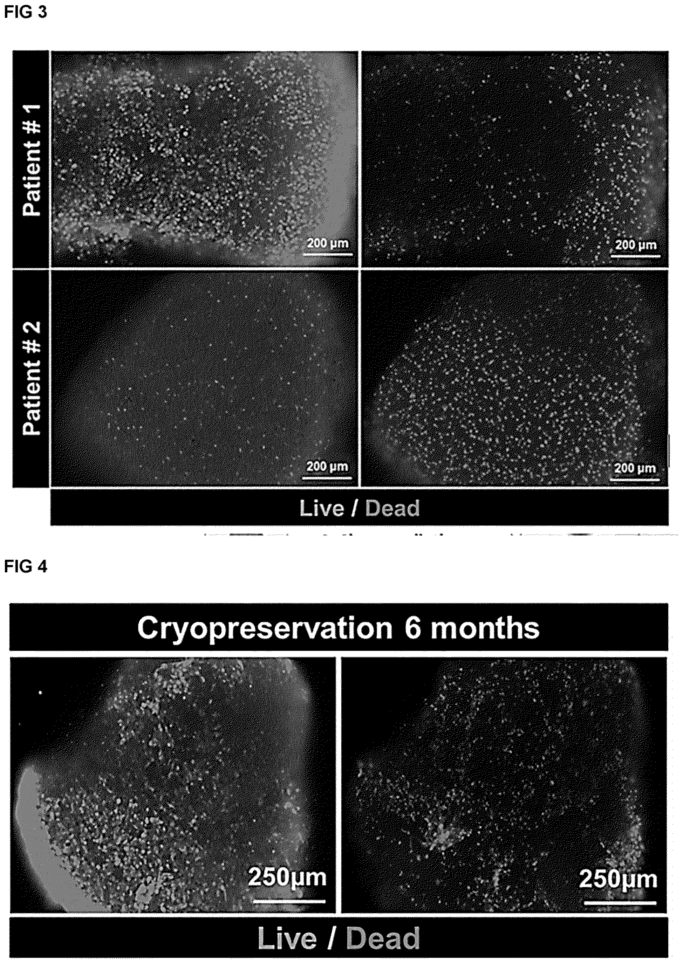

As a result of confirming the survival rate of the brain tumor tissue isolated from brain tumor patients using the Live/Dead kit, it could be seen that most brain tumor cells exhibited green fluorescence, indicating that there were many live cells (FIG. 3).

In addition, as a result of confirming the survival rate of the isolated brain tumor tissue after cryopreservation for 6 months using the Live/Dead kit, it could be confirmed that many brain tumor cells exhibited green fluorescence, indicating that the tissue survival rate was maintained even after cryopreservation (FIG. 4).

### 2. Production of brain tumor tissue model

The present inventors adjusted the molecular weight and solution concentration of PNIPAM to produce hydrogels and microchannels suitable for brain tumor tissue culture.

Specifically, to make microchannels of a certain thickness, spun fibers were manufactured from PNIPAM with molecular weights of ~40,000 and 85,000 and the diameters of the fibers were confirmed.

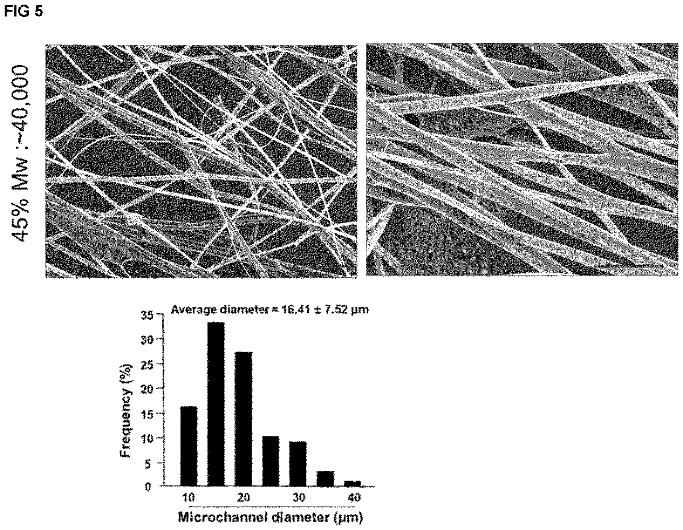

As a result, it was found that the average diameter of the PNIPAM fibers with a molecular weight of ~40,000 was 16.41 ± 7.52 µm, and fibers with a maximum diameter of 40 µm were also present, indicating that there was a large variation in the diameter among the fibers (FIG. 5).

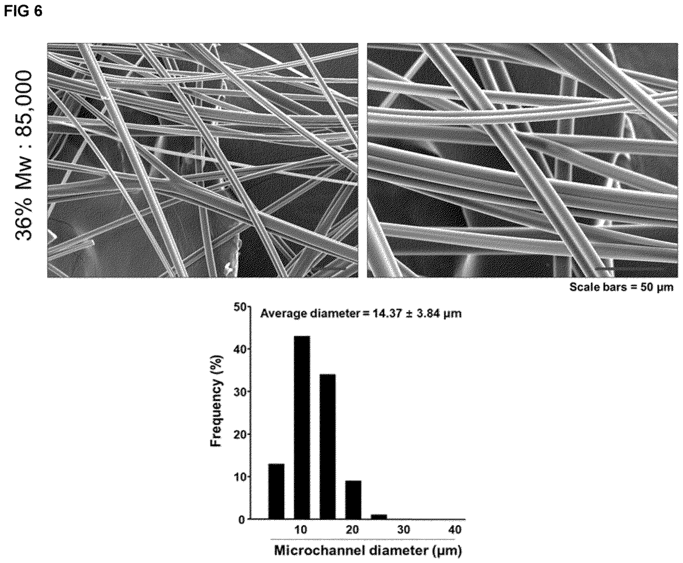

On the other hand, it was found that the average diameter of the PNIPAM fibers with a molecular weight of 85,000 was 14.37 ± 3.84 µm, and the maximum diameter of the fibers was also less than or equal to 25 µm, confirming that the diameters of the fibers were relatively uniform (FIG. 6).

Based on the above results, PNIPAM with a molecular weight of 85,000 was selected as PNIPAM suitable for the brain tumor tissue model. Next, the solution concentration of PNIPAM suitable for manufacturing spun fibers was confirmed. PNIPAM with a molecular weight of 85,000 was dissolved in methanol at concentrations of 32%, 33%, 34%, 35%, 36%, and 37% w/v%, respectively, and the resulting solutions were spun to produce PNIPAM fiber bundles.

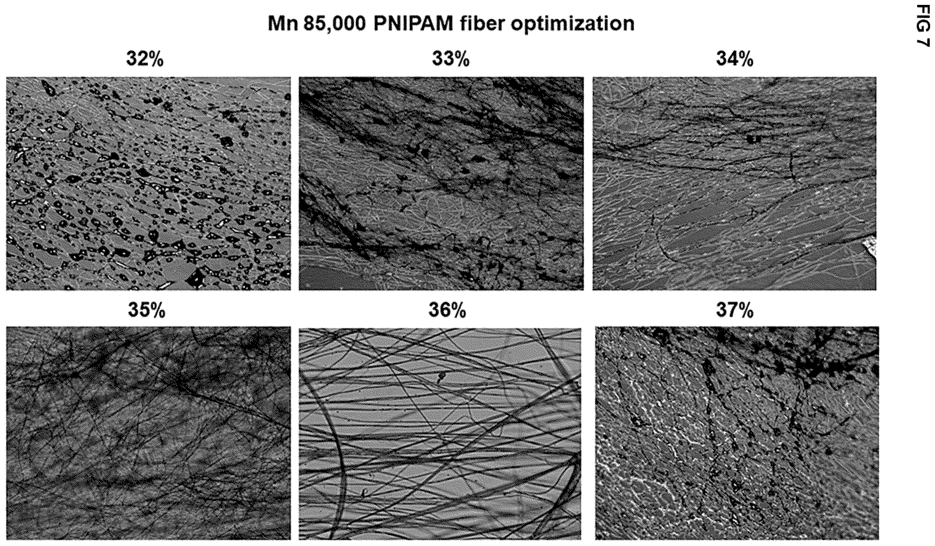

As a result of observing the produced fiber bundles, it could be seen that normal PNIPAM fibers were not formed at concentrations of 32%, 33%, and 37%, and PNIPAM fibers were formed at concentrations of 34% to 36%. In particular, PNIPAM fibers with even thicknesses and smooth surfaces were produced at a concentration of 36% (FIG. 7).

Since PNIPAM fibers produced under the above conditions exhibit a lower critical solution temperature (LCST) at around 32 °C, when producing an *ex vivo* brain tumor tissue model, the temperature maintenance ability was increased compared to existing low-molecular weight products, and the holding power to suppress the pushing phenomenon of the channel structure, which occurs during hydrogel injection, could be obtained. In addition, a vascular network structure suitable for tissue culture was developed by controlling the density of the produced microchannels.

### 3. Brain tumor tissue culture

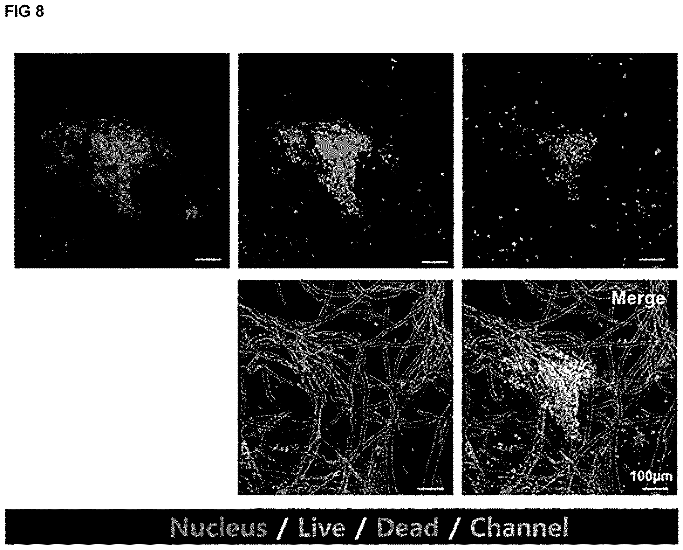

The brain tumor tissue in the brain tumor tissue model of the present invention was cultured for three days, and the tissue survival rate was confirmed. As a result, it was confirmed that most cells were live, and only a small number of cells were dead (FIG. 8).

To determine the differences between culturing brain tumor tissue on a brain tumor chip and other culture methods, the brain tumor tissue was cultured under various culture conditions. As a result of the culture, it could be seen that the cell survival rate was significantly superior when the brain tumor tissue was cultured on the brain tumor chip compared to other culture methods (FIG. 9).

In addition, when the brain tumor tissue was cultured on a brain tumor chip, brain cancer cells entering the vascular network structure could be confirmed (FIG. 10).

Brain cancer cells metastasize and migrate through blood vessels and nerves, and due to this migration ability of brain cancer cells, brain cancer is difficult to treat and has high recurrence and mortality rates. The results shown in FIG. 10 mean that the tumor tissue model of the present invention may reproduce the *in vivo* characteristics of highly mobile brain cancer cells at the laboratory level.

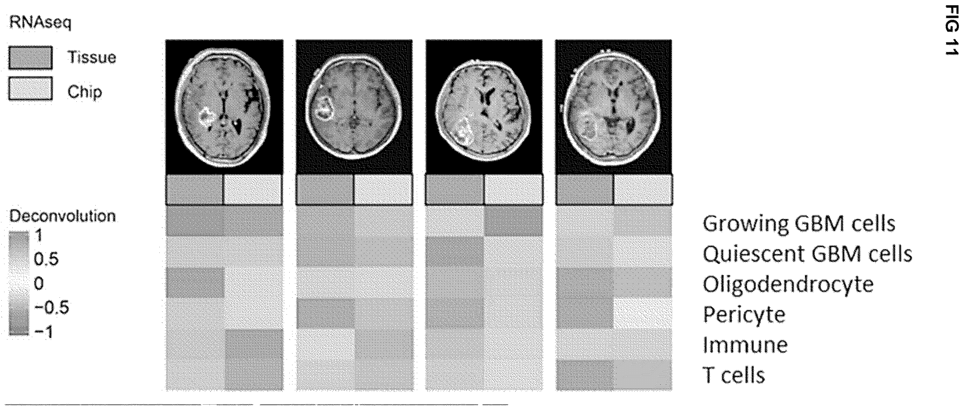

Gene expression patterns were compared in the form of a heatmap between the original brain tumor tissue (uncultured brain tumor tissue) and the brain tumor tissue cultured in the tumor tissue model. As a result, it was confirmed that there was no significant difference in the gene expression pattern of the brain tumor tissue ("Growing GBM cells" and "Quiescent GBM cells" in FIG. 11). These results mean that the original gene expression pattern may be maintained when the brain tumor tissue is cultured in the tumor tissue model (FIG. 11).

In FIG. 11, "Tissue" represents the original brain tumor tissue derived from the patient, and "Chip" represents the brain tumor tissue cultured in the tumor tissue model. In addition, "Growing GBM cells" refer to proliferating GBM cells, and "Quiescent GBM cells" refer to GBM cells at rest.

In addition, as a result of analyzing the expression pattern of the migratory genes according to culture time in the brain tumor tissue cultured in the tumor tissue model, it was confirmed that the expression of the migratory genes gradually increased as the culture time increased (FIG. 12). In FIG. 12, "Neuron-like" refers to nerve-derived cancer stem cells, and "Astrocyte-like" refers to astrocytes. In normal brain tissue, stem cells are highly migratory and play a role in replenishing damaged brain cells. However, when GBM occurs, stem cells turn cancerous and become neurons that have the characteristics of both cancer and stem cells. Astrocytes are mainly present in brain blood vessels and serve as bridges that supply nutrients from blood vessels to neurons. When these cells become cancerous, their mobility increases along blood vessels, which is a major cause of GBM recurrence and metastasis.

The results shown in FIG. 12 mean that the brain cancer cells in brain tumor tissue may move using the vascular network structure surrounding the tissue. Through the results shown in FIG. 12, it was confirmed that the tumor tissue model is a technology that simulates the metastatic characteristics of brain tumors.

## Claims

1. A tumor tissue model comprising:
a structure for tissue culture made of a biocompatible polymer and including a three-dimensional network formed by connecting a plurality of microchannels therein; and
tumor tissue isolated from the living body,
wherein the tumor tissue is encapsulated in the structure for tissue culture.

2. The tumor tissue model according to claim 1, wherein the biocompatible polymer is selected from the group consisting of gelatin, alginate, polyethylene glycol, polydimethylsiloxane, collagen, chitin, chitosan, keratin, cellulose, fibronectin, elastin, fibrinogen, fibromodulin, laminin, tenacin, vitronectin, hyaluronic acid, silk protein, silk fibroin and derivatives thereof, agarose, polylactic acid, polyglycolic acid, copolymers of polylactic acid and polyglycolic acid, polycaprolactone, poly(poly(ethylene oxide)terephthalate-co-butylene terephthalate, polyphosphoester, polyphosphazene, polyanhydride, polyorthoester, and polypropylene fumarate) diacrylate.

3. The tumor tissue model according to claim 1, wherein the tumor tissue is selected from the group consisting of brain tumors, stomach cancer, liver cancer, colon cancer, breast cancer, kidney cancer, prostate cancer, cervical cancer, endometrial cancer, and ovarian cancer.

4. The tumor tissue model according to claim 3, wherein the brain tumor is glioblastoma.

5. The tumor tissue model according to claim 1, wherein the structure for tissue culture further includes an inflow channel and an outflow channel which are connected to the microchannels and through which a fluid flows.

6. The tumor tissue model according to claim 1, wherein the tumor tissue has a length, width, and height of less than 1 mm.

7. The tumor tissue model according to claim 1, wherein the microchannel has a diameter of 5 to 50 µm.

8. A method for producing the tumor tissue model of claim 1, the method comprising:
(a) supplying a hydrogel solution containing tumor tissue to a mold containing a three-dimensional fiber bundle;
(b) crosslinking the hydrogel solution; and
(c) removing the three-dimensional fiber bundle from the crosslinked hydrogel.

9. The method according to claim 8, wherein the three-dimensional fiber bundle has a diameter of 5 to 50 µm.

10. The method according to claim 8, wherein the three-dimensional fiber bundle consists of a stimuli-responsive polymer selected from the group consisting of poly(N-isopropyl acrylamide), ethylene-co-vinyl acetate, ethylene-co-vinyl alcohol, poly(2-hydroxyethyl methacrylate), chitosan-poly(ethylene oxide), poly(acrylic acid)-poly(ethylene oxide), poly(2-hydroxyethyl methacrylate), poly(acrylamide-co-maleic acid), poly-N-vinylcaprolactam, poly(methacrylic acid), hydroxypropyl cellulose, polyvinyl methyl ether, poly(2-hydromethacrylic acid), poly(N-isopropylacrylamide), poly(N,N-diethylacrylamide), poly(N-diethyl methacrylamide), poly(methyl vinyl ether), poly(2-ethoxyethyl vinyl ether), poly(N-vinyl caprolactam), poly(N-vinyl isobutylamine), poly(N-vinyl-n-butylamine), and polyfumaric acid.

11. The method according to claim 10, wherein the poly(N-isopropyl acrylamide) has a number average molecular weight of 85,000.

12. The method according to claim 8, wherein the three-dimensional fiber bundle is prepared by dissolving poly(N-isopropyl acrylamide) having a number average molecular weight of 85,000 in methanol at a concentration of 34% to 36% and then spinning the solution.

13. The method according to claim 8, wherein the (c) is performed by converting the three-dimensional fiber bundle into a sol.

14. A tumor tissue culture method, comprising:
supplying a cell culture medium to the tumor tissue model of claim 1; and
culturing the tumor tissue model.

15. The tumor tissue culture method according to claim 14, wherein the cell culture medium is supplied at a rate of 1 to 15 mm/s.

16. The tumor tissue culture method according to claim 14, wherein the tumor tissue is brain tumor tissue.
